# EUROPEAN PATENT SPECIFICATION

(11) **EP 1 503 811 B1**
(45) Date of publication and mention of the grant of the patent: **31.10.2007**
(21) Application number: 02735983.5
(22) Date of filing: 09.05.2002
(51) Int. Cl.: A61L 11/00, B02C 19/11, B09B 3/00, B01J 19/12, H05B 6/80

(54) **DEVICE FOR THE STERILIZATION OF HOSPITAL WASTE**
ANLAGE ZUR STERILISIERUNG VON KRANKENHAUSABFALL
DISPOSITIF POUR LA STERILISATION DE DECHETS HOSPITALIERS

(30) Priority: 05.02.2002 IT PD20020027
(43) Date of publication of application: 09.02.2005
(73) Proprietor: Econos S.R.L., 45100 Rovigo (IT)
(72) Inventor: DEL BO, Massimo, I-45100 Rovigo (IT)
(74) Representative: Gustorf, Gerhard
(86) International application number: PCT/IT2002/000311
(87) International publication number: WO 2003/066105

(56) References cited:
- EP-A- 0 393 231
- EP-A- 0 649 661
- DE-C- 4 128 854
- US-A- 5 429 799

## Description

The present invention concerns devices for the sterilization of potentially infected hospital waste.

Hospital waste cannot be directly disposed of in dumps, like common solid urban waste, due to its high degree of infectiveness.

At present it is compulsory to incinerate hospital waste in special systems and the health structures have to bear all the direct and indirect expenses connected to these treatments.

In any case, there always is the risk of infection following the transport to the incinerator and other tranfers carried out by the personnel in charge, unless suitable and particularly expensive countermeasures are adopted.

Hospital waste, closed in special containers in order to protect the personell that must handle it, comprises heterogeneous material, like gauze and tampons, and closed elements, like syringes and bottles, that prevent sterilizing substances from having the desired effect on each single part of the waste.

The known sterilization equipment and devices, like for example the UV rays irradiating equipment, do not ensure the necessary sterilization of hospital waste, since this type of equipment is developed and constructed for the treatment of homogeneous and separate material.

The studies carried out and the measures and solutions adopted up to now have not led to any acceptable result.

A device as set forth in the preamble of claim 1 is known from EP-A 649 661. In this known device, sterilization is effected only in the oven (sterilization chamber) when its valve is closed.

The top of the hopper and consequently the preheating device housing the screw feeder remain always open to the outside ambient. During the sterilization process in the oven, the shredded waste remains as a compact mass in the oven.

From EP-A 393 231 a similar device is known in which the shredded waste is fed by a conveyor to a microwave chamber and then through a further conveyor into a warming chamber.

DE-C 41 28 854 discloses a hospital refuse sterilization plant in which a hopper, a triturator and a mixing chamber are combined in a single vertical unit. The mixing chamber is only provided with steam inlet means without any microwave devices. The mixing chamber has an agitating device having U-shaped wings moving near the inner wall of the mixing chamber. Said agitating device has a vertical rotation axis. Due to that construction, the mixing effect is limited to the zone of the lateral wall whereas the material in the centre is not moved by the wings.

In order to overcome the drawbacks described above, a device for the sterilization of hospital waste has been studied and implemented.

The aim of the hospital waste sterilization device is to ensure the complete sterilization of the waste, so that it can be successively disposed of as solid urban waste.

Another aim of the hospital waste sterilization device is to guarantee the absence of closed containers among the waste to be sterilized.

The device for the sterilization of hospital waste involves a first trituration of the waste introduced into it, so as to open the waste containers, break up the bigger objects, break and crush the containers. The triturated waste is sprinkled and mixed with high-temperature steam.

Finally, the mass of triturated waste is put into a hermetic container provided with a stirrer, where it is subjected to microwave irradiation and to a high-temperature pressurized steam treatment for a given lapse of time.

If, for any reason, during the process the temperature and/or pressure values lower below the minimum preset level, the whole microwave irradiation and steam flooding process will be started again from the beginning.

The combined action of steam and microwaves, together with the continuous stirring, sterilizes completely the whole mass of triturated waste.

The entire process, from the introduction into the triturator until the exit from the steam and microwave treatment container, takes place in an insulated environment, with no contact with the outside.

The characteristics of the hospital waste sterilization device will be highlighted in greater detail in the following description of one among many possible applications of the invention, with reference to the enclosed drawings.

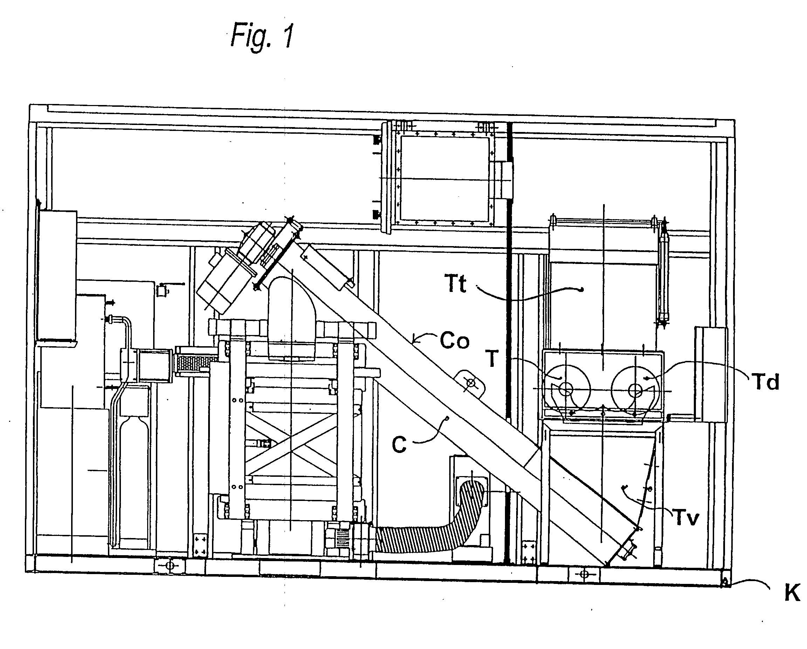

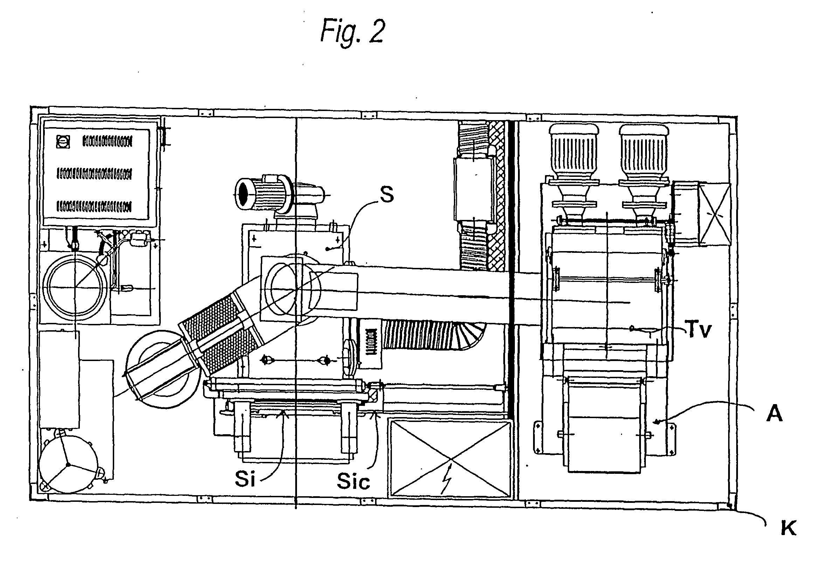

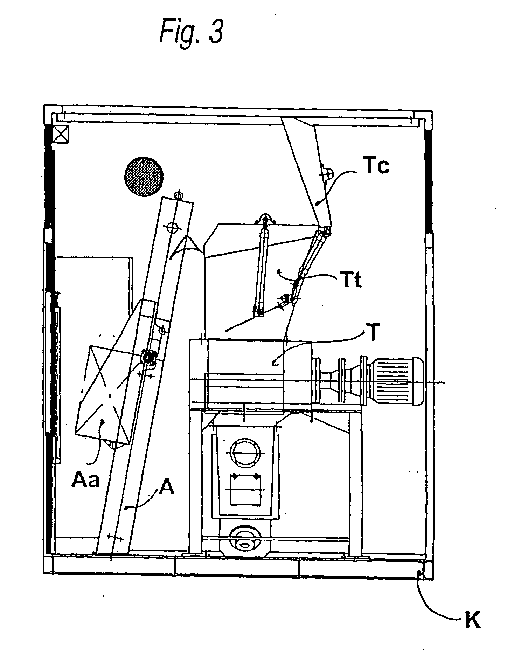

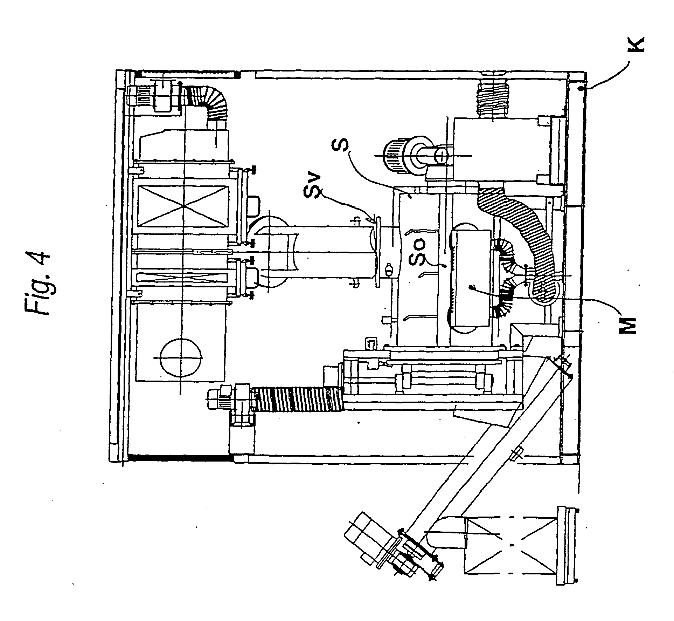

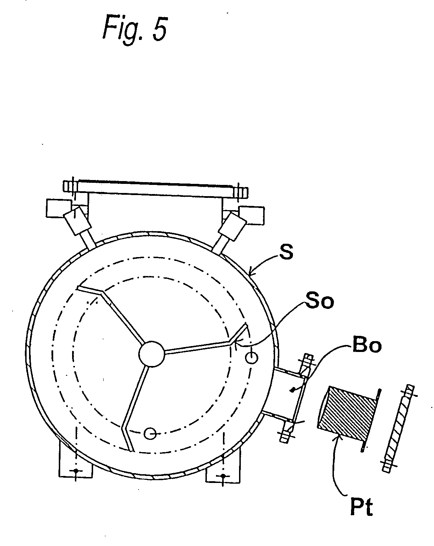

Figure 1 is a front view of the device, Figure 2 is a top view of the same, Figures 3 and 4 are side views, while Figure 5 shows a cross section of the sterilization chamber.

The figures schematically show the various parts of the new sterilization device, more precisely the loading device (A), the triturator (T) with the relevant hopper (Tt), the transport screw feeder (C), the sterilization chamber (S) and the microwave generators (M).

The loading device (A) is constituted by a basket or container (Aa) that receives the bags or containers of waste to be sterilized and then is lifted and overturned in the hopper (Tt) of the triturator (T), for example by means of chains or of a fluid-mechanic system.

The triturator (T) comprises a hopper (Tt) with a hermetic cover at the top (Tc) and a series of horizontal toothed drums (Td) positioned side by side at the bottom.

A thrusting device or another device to convey and thrust the waste towards the toothed drums (Td) may be provided inside the hopper (Tt) of the triturator (T), in such a way as to optimize the operation of the triturator (T) and ensure the correct trituration of the waste.

Said hopper (Tt) is kept under vacuum and the air present inside it is extracted, conveyed towards a so-called "absolute" filter and eliminated.

Under the toothed drums (Td) of the triturator (T) there is a collection compartment (Tv) for the temporary accumulation of triturated waste, provided with optical sensors or other types of sensors for the detection of the introduction of waste into the screw feeder (C) and the achievement of the optimal loading level.

The screw (C) is closed inside an inclined duct (Co) and connects the collection compartment (Tv) of the triturator (T) with the upper opening of the sterilization chamber (S). Along the duct (Co) of the screw feeder (C) there are nozzles suitable for introducing steam inside the duct (Co) itself.

The sterilization chamber (S) comprises a preferably cylindrical container provided with two openings, more precisely, an upper opening and another lateral or front opening.

The upper opening of the sterilization chamber (S) is provided with a gate valve (Sv) that separates it hermetically from the screw feeder (C).

Similarly, the lateral or front opening of the sterilization chamber (S) is provided with a mobile gate (Si) that opens and closes completely the mouth of the sterilization chamber (S).

In particular, the gate (Si) of the opening of the sterilization chamber (S) is supported by a carriage (Sic) that is translated horizontally on guides owing to the action of a preferably pneumatic cylinder. Said gate (Si) is translated in such a way as to close the opening of the sterilization chamber (S) by jacks (Sin).

Inside the sterilization chamber (S) there is a stirrer (So) preferably constituted by an horizontal shaft with radial arms or blades.

A rear steam inlet nozzle is positioned on the wall of the sterilization chamber (S), while the microwave generators (M) are arranged on one or more levels, around the sterilization chamber (S). The part (Bo) of the sterilization chamber wall in correspondence with said microwave generators (M) is constituted by elements (Pt) made of Teflon or other suitable materials, so as to obtain the best possible results from the irradiation process.

Optical, ultrasound or other sensors are positioned in the upper part of the sterilization chamber (S) to detect the maximum loading level of triturated waste in the sterilization chamber (S).

Other temperature and pressure sensors are placed in appropriate positions on the walls of the sterilization chamber (S), in such a way as to have a constant monitoring of temperature and pressure.

All the various electric, electronic and mechanical parts are controlled by an appropriate electronic circuit that isn't shown in the figure and supervises the operation of the new device for the sterilization of hospital waste.

All the steps of the process, from the introduction of the waste into the hopper (Tt) of the triturator (T) to the exit of the same from the sterilization chamber, take place along a closed circuit, with no contact, even aerial, between the waste and the outside.

The operator places one or more closed bags or containers with the waste to be sterilized in the basket or container (Aa) of the loading device (A) and starts the sterilization cycle of the new hospital waste sterilization device.

The loading device (A) lifts the basket or container (Aa) and overturns it into the hopper (Tt) of the triturator, introducing the closed bags or containers into the triturator (T) itself.

The sensors present in the upper part of the hopper (Tt) detect the fall of the waste and activate the closing of the hermetic cover (Tc) of the hopper (Tt).

Immediately afterwards the toothed drums (Td) of the triturator (T) start rotating, thus breaking up the waste and opening and breaking any hollow object such as bottles and syringes. At the same time the thrusting element, if present, compresses and/or conveys the waste towards said toothed drums (Td), in such a way as to optimize the trituration process.

The waste triturated by the toothed drums (Td) are first accumulated in the collection compartment (Tv) provided under the toothed drums (Td) and then taken by the screw feeder (C).

The screw feeder (C) conveys the triturated waste towards the mouth of the sterilization chamber (S). While moving along the screw (C) and the relevant duct (Co), the waste is sprinkled with steam through the nozzles present on the duct (Co) itself. In this way the screw feeder (C), besides transporting the waste towards the sterilization chamber (S), mixes it and allows the steam to penetrate among all the triturated parts of the waste.

At the end of the screw feeder (C) the waste, triturated and pretreated with steam, is introduced into the sterilization chamber (S) through the gate valve (Sv) provided on the upper opening of the sterilization chamber (S) itself.

As the waste is introduced into the sterilization chamber (S), the stirrer (So) present inside the sterilization chamber (S) itself distributes said waste homogeneously.

The gate valve (Sv) is closed as soon as the maximum loading level of the triturated waste is reached and at this point the actual sterilization phase inside the sterilization chamber (S) is started: the microwave generators (M) are set in operation and high-temperature pressurized steam is introduced into the sterilization chamber (S).

During the irradiation of the triturated waste with microwaves and the steam flooding process inside the sterilization chamber (S), the stirrer (So) keeps rotating, thus mixing the triturated waste in such a way as to obtain the maximum microwave irradiation and steam penetration into the triturated waste.

The action of the microwaves and the steam makes it possible to increase the temperature and pressure values inside the sterilization chamber (S). The action of the stirrer (So) allows the microwaves and the steam to reach all the particles of the triturated waste, thus ensuring that each single part of the waste mass reaches the same pressure and temperature levels reached by the rest of the triturated waste mass.

At the end of the sterilization phase, that is, when the triturated waste has been subjected to sufficient temperatures and pressures for a given lapse of time, the microwave generators (M) are stopped, the sterilization chamber (S) is depressurized and the gate (Si) of the sterilization chamber (S) itself is opened in order to allow the completely sterilized waste to be discharged.

This triturated and sterilized hospital waste is then passed on for the successive phase of the process, in which it is put into sacks in order to be delivered to the dump exactly like solid urban waste.

If the temperature and/or the pressure inside the sterilization chamber lower below the minimum level set, the electronic control circuit of the new device will make the entire sterilization cycle inside the sterilization chamber (S) start again from the beginning, until the required temperature and pressure values are maintained for the necessary lapse of time.

The hospital waste sterilization device offers considerable advantages and interesting characteristics:
- after the introduction of the closed waste bags inside the new device, there isn't any further contact between the waste and the operators, or between the waste and the environment outside the new device;
- the containers, like phials, syringes and bottles, are opened and broken up, so that the complete sterilization of all the waste is ensured;
- the stirrer inside the sterilization chamber mixes the waste fragments, so that they all reach the same temperature and are thus completely sterilized;
- complete sterilization is ensured by the electronic control circuit, which guarantees that the waste is subjected to a temperature exceeding the minimum temperature set;
- the sterilized waste produced by the new device can be treated as solid urban waste and can be delivered to normal dumps;
- the cost, the time required by the process and the risks for the personnel in charge with it are much lower than those involved by the known waste treatment systems;
- the device described above can be easily contained into standard-size containers and therefore is easy to transport and to install.

## Claims

1. Device for the sterilization of hospital waste, comprising a triturator (T) with a hopper (Tt) and toothed drums (Td) for breaking up the waste, a screw feeder (C) to convey the triturated waste from said triturator (T) into an airtight sterilization chamber (S) equipped with microwave generators (M), and a steam generator producing high temperature pressurized steam blowed through nozzles into said screw feeder (C) and into said sterilization chamber (S), wherein the waste closed in the sterilization chamber (S) is subjected to microwave irradiation and mixed with high-temperature steam, **characterized in that** said sterilization chamber (S) is equipped with an inner stirrer (So) for mixing its contents, said stirrer (So) comprising a horizontally rotating shaft to which arms or blades are fastened, and that the hopper (Tt) of said triturator (T) is provided at its top with a hermetic cover (Tc), such that during the whole sterilization process the waste treated in the triturator (T), the screw feeder (C) and the sterilization chamber (S) is completely insulated from the outside.

2. Device according to claim 1, wherein on the walls of the sterilization chamber (S), in correspondence with said microwave generators (M), there are holes (Bo) each of which is closed by a plug element (Pt) made of Teflon or other suitable materials.

3. Device according to claim 1 or 2, wherein all the electric, electronic and mechanical parts of said sterilization device are controlled by an electronic circuit controlling and supervising their operation, and wherein, if the temperature and pressure values inside the sterilization chamber (S) lower below the minimum levels set, said electronic circuit makes the treatment cycle inside the sterilization chamber (S) start again from the beginning.

4. Device according to any of the preceding claims, **characterized in that** it is contained in a modular frame or container easily to be transported and installed.

## Patentansprüche

1. Anlage zum Sterilisieren von Krankenhausabfällen, umfassend einen Schredder (T) mit einem Einfülltrichter (Tt) und Zahnwalzen (Td) zum Zerkleinern des Abfalls, einen Schneckenförderer (C) zum Transportieren des zerkleinerten Abfalls von dem Schredder (T) in eine luftdichte Sterilisationskammer (S), die mit Mikrowellengeneratoren (M) ausgerüstet ist, sowie einen Dampferzeuger zur Erzeugung von Druckdampf hoher Temperatur, der durch Düsen in den Schneckenförderer (C) und die Sterilisationskammer (S) geblasen wird, wobei der in der Sterilisationskammer (S) eingeschlossene Abfall einer Mikrowellenbestrahlung ausgesetzt ist und mit dem Hochtemperaturdampf gemischt wird, **dadurch gekennzeichnet, dass** die Sterilisationskammer (S) mit einem inneren Rührwerk (So) zum Durchmischen ihres Inhalts ausgerüstet ist, wobei das Rührwerk (So) eine horizontale Drehwelle hat, an der Arme oder Schaufeln befestigt sind, und dass der Einfülltrichter (Tt) des Schredders (T) an seinem oberen Ende einen hermetischen Abschlußdeckel (Tc) aufweist, so dass während des gesamten Sterilisationsprozesses der in dem Schredder (T) behandelte Abfall, der Schneckenförderer (C) und die Sterilisationskammer (S) nach außen vollständig abgedichtet sind.

2. Anlage nach Anspruch 1, **dadurch gekennzeichnet, dass** die Wände der Sterilisationskammer (S) im Bereich der Mikrowellengeneratoren (M) Öffnungen (Bo) haben, von denen jede durch einen Stopfen (Pt) aus Teflon oder anderen geeigneten Werkstoffen abgeschlossen ist.

3. Anlage nach Anspruch 1 oder 2, **dadurch gekennzeichnet, dass** alle elektrischen, elektronischen oder mechanischen Teile der Sterilisationsanlage von einer elektronischen Schaltung gesteuert werden, die deren Funktion steuert und überwacht und die bei Abfall der Temperatur und des Druckes in der Sterilisationskammer (S) unter einen eingestellten Minimalwert den Behandlungszyklus in der Sterilisationskammer (S) von Beginn an neu startet.

4. Anlage nach einem der vorhergehenden Ansprüche, **dadurch gekennzeichnet, dass** diese in einem modularen Rahmen oder Behälter untergebracht ist, der leicht zu transportieren und installieren ist.

## Revendications

1. Installation pour stériliser les déchets des hôpitaux, comprenant un triturateur (T) avec une trémie d'alimentation (Tt) et des calandres dentées (Td) pour broyer les déchets, une vis de transfert (C) pour transporter les déchets broyés du triturateur (T) à une chambre étanche de stérilisation (S) équipée de générateurs à micro-ondes (M), et un générateur de vapeur pour produire de la vapeur sous pression à haute température, pression qui est injectée par des buses dans la vis de transfert (C) et dans la chambre de stérilisation (S), les déchets contenus dans la chambre de stérilisation (S) étant soumis à des micro-ondes et mélangés avec la vapeur à haute température, **caractérisée par le fait que** la chambre de stérilisation (S) est équipée d'un malaxeur interne (So) pour mélanger son contenu, ledit malaxeur (So) ayant un arbre horizontal de rotation auquel sont fixées des bras ou des pales, et **par le fait que** la trémie d'alimentation (Tt) du triturateur (T) est muni à son extrémité supérieure d'un couvercle (Tc) pour le recouvrir hermétiquement, de sorte que, durant toute la durée du processus de stérilisation, les déchets contenus dans le triturateur (T), la vis de transfert (C) et la chambre de stérilisation (S) soient hermétiquement isolés de l'extérieur.

2. Installation selon la revendication 1 **caractérisée par le fait que** les parois de la chambre de stérilisation (S) sont munie d'ouvertures (Bo) dans la zone des générateurs à micro-ondes (M), chaque ouverture étant close par un bouchon (Pt) en téflon ou autres matériaux adéquats.

3. Installation selon la revendication 1 ou 2 **caractérisée par le fait que** tous les éléments électriques, électroniques ou mécaniques de l'installation de stérilisation sont pilotés par un circuit électronique apte à commander et à surveiller les fonctions de l'installation et qui remet le cycle en route de traitement dès que la température et la pression dans la chambre de stérilisation (S) redescendent au-dessous d'une valeur minimum préréglée.

4. Installation selon une quelconque des revendications précédentes **caractérisée par le fait que** l'installation est logée dans un cadre ou un conteneur modulaire facile à transporter et à installer.
